(19) Europäisches Patentamt / European Patent Office / Office européen des brevets

(11) **EP 4 497 338 A1**

(12) **EUROPEAN PATENT APPLICATION**
published in accordance with Art. 153(4) EPC

(43) Date of publication:
29.01.2025  Bulletin 2025/05

(21) Application number: 23784211.7

(22) Date of filing: 29.03.2023

(51) International Patent Classification (IPC):
*A24F 40/40* (2020.01)

(52) Cooperative Patent Classification (CPC):
**A24F 40/40; A24F 40/46; A24F 40/51; A24F 40/53; A24F 40/57**

(86) International application number:
**PCT/CN2023/084846**

(87) International publication number:
**WO 2023/193647 (12.10.2023 Gazette 2023/41)**

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR

(30) Priority: 07.04.2022  CN 202210361280

(71) Applicant: Shenzhen First Union Technology Co., Ltd.
Shenzhen, Guangdong 518000 (CN)

(72) Inventors:
• FAN, Long
Shenzhen, Guangdong 518000 (CN)
• LONG, Zhiming
Shenzhen, Guangdong 518000 (CN)
• YU, Peixia
Shenzhen, Guangdong 518000 (CN)
• XU, Zhongli
Shenzhen, Guangdong 518000 (CN)
• LI, Yonghai
Shenzhen, Guangdong 518000 (CN)

(74) Representative: Ran, Handong et al
Maucher Jenkins
Seventh Floor Offices
Artillery House
11-19 Artillery Row
London SW1P 1RT (GB)

(54) **AEROSOL GENERATING APPARATUS AND METHOD FOR COUNTING NUMBER OF PUFFS OF USER**

(57)    An aerosol generating apparatus and a method for detecting puffs of a user. The apparatus comprises a heater (2), which is used for heating an aerosol generating product (1) so as to generate an aerosol for a user to puff; a power source (3), which is electrically connected to the heater (2), so that the temperature on the heater (2) reaches a target temperature corresponding to each puff of the user; and a controller (6), which is connected to the heater (2). The controller (6) is used to detect the actual temperature of the heater (2) during an operating process of the aerosol generating apparatus, determine a temperature change speed, add 1 to the number of puffs of the user each time the temperature change speed meets a preset condition, and record the number of puffs, wherein the temperature change speed is a change amount of the actual temperature of the heater (2) relative to the target temperature per unit time.

FIG. 4

Processed by Luminess, 75001 PARIS (FR)

EP 4 497 338 A1

# EP 4 497 338 A1

**Description**

**CROSS-REFERENCE TO RELATED APPLICATIONS**

This application claims priority to Chinese Patent Application No. 202210361280.3, filed with the China National Intellectual Property Administration on April 7, 2022 and entitled "AEROSOL GENERATING APPARATUS AND METHOD FOR COUNTING NUMBER OF PUFFS OF USER", which is incorporated herein by reference in its entirety.

**TECHNICAL FIELD**

Embodiments of this application relate to the field of aerosol generating technologies, and in particular, to an aerosol generating apparatus and a method for counting a number of puffs of a user.

**BACKGROUND**

An aerosol generating apparatus includes a heater and a power source. The heater is configured to heat an aerosol generating product, so as to generate an aerosol. The power source is connected to the heater, so as to provide the heater with power required for heating. The heater is at a sufficient temperature to enable the aerosol generating product to generate sufficient aerosols.

However, during puffing, a change in an airflow flowing through the heater reduces the temperature of the heater. Therefore, a user puffing event may be determined by detecting the temperature of the heater.

In some existing solutions, the change in the airflow flowing through the heater is determined only by detecting a difference between the temperature and a target temperature, to determine the user puffing event. However, this manner may be directly affected by setting of the target temperature, resulting in erroneous determining of the puffing event.

**SUMMARY**

Embodiments of this application provide an aerosol generating apparatus and a method for counting a number of puffs of a user. Through a temperature change speed of an actual temperature of a heater relative to a target temperature, the number of puffs of the user is counted, so as to improve accuracy of the counting.

An embodiment of this application provides an aerosol generating apparatus, including:

a heater, configured to heat an aerosol generating product, so as to generate an aerosol for a user to inhale;

a power source, electrically connected to the heater, to cause a temperature on the heater to reach a target temperature corresponding to each puff of a user; and

a controller, connected to the heater.

The controller is configured to: during operation of the aerosol generating apparatus, detect an actual temperature of the heater, and determine a temperature change speed; determine whether the temperature change speed meets a preset condition; and add 1 to a number of puffs of the user each time the temperature change speed meets the preset condition, and record the number of puffs.

The temperature change speed is a change amount of the actual temperature of the heater relative to the target temperature per unit time.

Optionally, the controller is configured to determine whether the temperature change speed is greater than a first threshold, and add 1 to the number of puffs of the user each time the temperature change speed is greater than the first threshold.

Optionally, the controller is configured to: determine an acceleration of the temperature change speed; determine whether the acceleration is greater than a second threshold; and add 1 to the number of puffs of the user each time the acceleration is greater than the second threshold.

The acceleration is a change amount of the temperature change speed per unit time.

Optionally, the controller is configured to: determine a difference $\Delta T1$ between the target temperature and the actual temperature of the heater when the temperature change speed is equal to a first threshold; determine a difference $\Delta T2$ between the target temperature and the actual temperature of the heater when an acceleration of the temperature change speed is equal to a second threshold; determine an absolute change amount $\Delta$ based on $\Delta T2$ and $\Delta T1$; determine whether the absolute change amount $\Delta$ is greater than a third threshold; and add 1 to the number of puffs of the user each time the absolute change amount $\Delta$ is greater than the third threshold.

Optionally, the absolute change amount $\Delta = \Delta T2 - \Delta T1$.

Optionally, a first threshold of an $(N+M)^{th}$ puff of the user is less than a first threshold of an $N^{th}$ puff of the user, N is

2

an integer greater than or equal to 2, and M is an integer greater than or equal to 1; and/or
a third threshold of the (N+M)th puff of the user is less than a third threshold of the Nth puff of the user, N is an integer greater than or equal to 2, and M is an integer greater than or equal to 1.

Optionally, the first threshold and/or the third threshold gradually decreases as the number of puffs of the user increases.

Optionally, when the acceleration is equal to the second threshold, the temperature change speed reaches a maximum.

Optionally, the controller is connected to the power source, and is configured to control the power source to perform power compensation on the heater when the acceleration is equal to the second threshold, to cause the heater to reach the corresponding target temperature.

Optionally, a target temperature of an (N+M)th puff of the user is less than a target temperature of an Nth puff of the user, N is an integer greater than or equal to 2, and M is an integer greater than or equal to 1.

Optionally, the target temperature continuously decreases as the number of puffs of the user increases.

Optionally, the target temperature remains unchanged as the number of puffs of the user increases.

An embodiment of this application provides a method for counting a number of puffs of a user by an aerosol generating apparatus. The aerosol generating apparatus includes a heater, a power source configured to supply power to the heater, and a controller. The method includes:

detecting an actual temperature of the heater;
determining a temperature change speed of the actual temperature of the heater relative to a target temperature, where the target temperature is a preset target temperature corresponding to each puff of the user, and the temperature change speed is a change amount of the actual temperature of the heater relative to the target temperature per unit time;
determining whether the temperature change speed meets a preset condition;
adding 1 to the number of puffs of the user each time the temperature change speed meets the preset condition; and
recording the number of puffs.

Optionally, the determining whether the temperature change speed meets a preset condition, and adding 1 to the number of puffs of the user each time the temperature change speed meets the preset condition includes:

determining whether the temperature change speed is greater than a first threshold; and
adding 1 to the number of puffs of the user each time the temperature change speed is greater than the first threshold.

Optionally, the method further includes:
determining an acceleration of the temperature change speed, where the acceleration is a change amount of the temperature change speed per unit time.

The determining whether the temperature change speed meets a preset condition, and adding 1 to the number of puffs of the user each time the temperature change speed meets the preset condition includes:

determining whether the temperature change speed is greater than a second threshold; and
adding 1 to the number of puffs of the user each time the acceleration of the temperature change speed is greater than the second threshold.

Optionally, the method further includes:

determining a difference $\Delta T1$ between the target temperature and the actual temperature of the heater when the temperature change speed is a first threshold;
determining an acceleration of the temperature change speed, where the acceleration is a change amount of the temperature change speed per unit time;
determining a difference $\Delta T2$ between the target temperature and the actual temperature of the heater when the acceleration is greater than a second threshold; and
determining an absolute change amount $\Delta$ of the actual temperature of the heater relative to the target temperature, where the absolute change amount is equal to $\Delta T2-\Delta T1$.

The adding 1 to the number of puffs of the user each time the temperature change speed meets the preset condition includes:

determining whether the absolute change amount is greater than a third threshold; and

adding 1 to the number of puffs of the user each time the absolute change amount is greater than the third threshold.

Optionally, during puffing of the user, the preset target temperature does not remain unchanged.

An embodiment of this application provides a non-volatile computer-readable storage medium, storing computer-executable instructions. The computer-executable instructions, when executed by a controller, cause the controller to perform the method described above.

An embodiment of this application further provides a computer program product, including a computer program stored in a non-volatile computer-readable storage medium. The computer program includes program instructions. The program instructions, when executed by a controller, cause the controller to perform the method described above.

According to the aerosol generating apparatus and the method for counting a number of puffs of a user described above, during operation of the aerosol generating apparatus, the controller determines the temperature change speed, that is, a change amount of the actual temperature of the heater per unit time relative to a target temperature at a current stage based on the set target temperature by detecting the actual temperature of the heater, determines whether the temperature change speed meets the preset condition, adds 1 to the number of puffs of the user each time the temperature change speed meets the preset condition, and records the number of puffs. The number of puffs of the user is determined through the temperature change amount, which is simple, convenient, and highly precise. Especially during the operation of the aerosol generating apparatus, when the set target temperature changes with puffing time, the number of puffs is identified through the temperature change speed, which may avoid being affected by a change of the target temperature, and help accurately identify the number of puffs.

BRIEF DESCRIPTION OF THE DRAWINGS

One or more embodiments are exemplarily described with reference to pictures in accompanying drawings corresponding to the embodiments, and the exemplary descriptions do not constitute a limitation on the embodiments. Elements in the accompanying drawings that have same reference numerals are represented as similar elements, and unless otherwise particularly stated, the pictures in the accompanying drawings are not drawn to scale.

FIG. 1 is a schematic diagram of an aerosol generating apparatus according to an embodiment of this application.
FIG. 2 is a schematic diagram of a circuit of an aerosol generating apparatus according to an embodiment of this application.
FIG. 3 is a graph showing an ideal state of a target of a heater during puffing of a user according to an embodiment of this application.
FIG. 4 is a graph showing an actual state of a target temperature of a heater during puffing of a user according to an embodiment of this application.
FIG. 5 is a graph showing a change rate of a difference between a target temperature and a temperature of a heater within a detection period according to another embodiment of this application.
FIG. 6 is a graph of a temperature change speed as a function of time within a detection period according to another embodiment of this application.
FIG. 7 is a graph of an acceleration as a function of time within a detection period according to another embodiment of this application.
FIG. 8 is a flowchart of a method for counting a number of puffs according to an embodiment of this application.
FIG. 9 is a flowchart of a method for counting a number of puffs according to another embodiment of this application.
FIG. 10 is a flowchart of a method for counting a number of puffs according to another embodiment of this application.
FIG. 11 is a flowchart of a method for counting a number of puffs according to another embodiment of this application.
FIG. 12 is a flowchart of a method for counting a number of puffs according to another embodiment of this application.
FIG. 13 is a flowchart of a method for counting a number of puffs according to another embodiment of this application.

In the drawings:

1. Aerosol generating product;
2. Heater;
3. Power source;
4. Insertion detector;
5. User interface;
6. Controller;
8. Temperature sensor.

## DETAILED DESCRIPTION

Technical solutions in embodiments of this application are clearly and completely described below with reference to accompanying drawings in the embodiments of this application. Apparently, the described embodiments are merely some rather than all of the embodiments of this application. All other embodiments obtained by a person of ordinary skill in the art based on the embodiments of this application without creative efforts shall fall within the protection scope of this application.

Terms "first", "second", and "third" in this application are merely intended for a purpose of description, and shall not be understood as an indication or implication of relative importance or implicit indication of a quantity or an order of indicated technical features. All directional indications (for example, up, down, left, right, front, and back) in the embodiments of this application are only used for explaining relative position relationships, movement situations, or the like among the various components in a specific posture (as shown in the accompanying drawings). If the specific posture changes, the directional indication changes accordingly. In addition, terms "include", "have", and any variant thereof are intended to cover a non-exclusive inclusion. For example, a process, a method, a system, a product, or a device that includes a series of steps or units is not limited to the listed steps or units, and instead, further optionally includes a step or unit that is not listed, or further optionally includes another step or unit that is intrinsic to the process, the method, the product, or the device.

The "embodiment" mentioned in the specification means that particular features, structures, or characteristics described with reference to the embodiments may be included in at least one embodiment of this application. The phrase appearing at various locations in this specification does not necessarily refer to a same embodiment, and is not an independent or alternative embodiment mutually exclusive of another embodiment. A person skilled in the art explicitly or implicitly understands that the embodiments described in the specification may be combined with other embodiments.

It should be noted that, when an element is referred to as "being fixed to" another element, the element may be directly located on the another element, or an intermediate element may exist. When an element is considered to be "connected to" another element, the element may be directly connected to the another element, or one or more intermediate elements may simultaneously exist between the element and the another element. Terms "vertical", "horizontal", "left", "right", and similar expressions used in this specification are only for purposes of illustration, and do not represent a unique implementation.

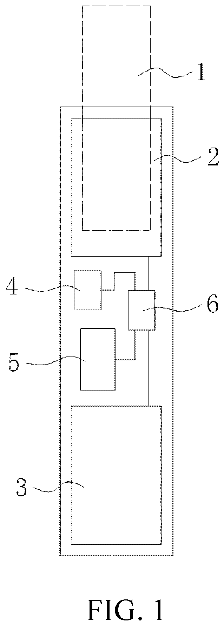

An embodiment of this application provides an aerosol generating apparatus. The apparatus may be configured to heat an aerosol generating product, so that the aerosol generating product volatilizes an aerosol for smoking. The aerosol may include Chinese herbal medicine, nicotine, or a flavor substance such as a tobacco flavor. In an embodiment shown in FIG. 1, an aerosol generating product 1 is a tobacco product (for example, a cigarette or a cigar), which is not limited.

In the embodiment shown in FIG. 1, an aerosol generating apparatus includes a receiving cavity configured to receive the aerosol generating product 1 and a heater 2 configured to heat the aerosol generating product 1, and further includes a power source 3. The power source 3 is configured to supply power to the heater 2 for operation.

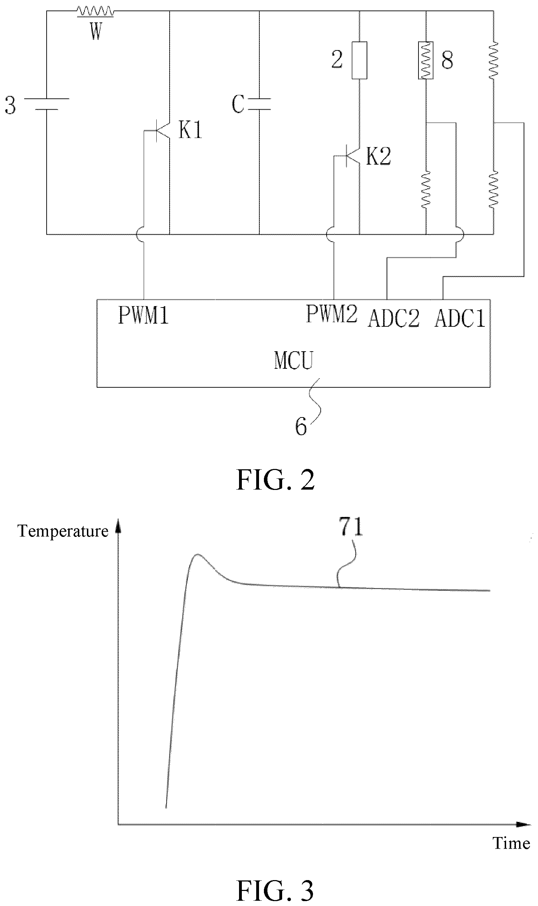

Referring to FIG. 1 and FIG. 2, the aerosol generating apparatus has a socket, and the aerosol generating product 1, for example, a cigarette, is removably received in the receiving cavity through the socket. At least part of the heater 2 extends in a longitudinal direction in the receiving cavity, and generates heat through electromagnetic induction under a changing magnetic field, or generates heat through resistance when being energized, or radiates an infrared ray to the aerosol generating product when being excited, so that the aerosol generating product 1, for example, a cigarette, is heated, and at least one component of the aerosol generating product 1 is volatilized, to form an aerosol for inhalation. The power source 3 includes a battery core. The battery core is a rechargeable direct current battery core, and may output a direct current. In another embodiment, the battery core may alternatively be a disposable battery, which is not rechargeable or does not need to be charged. In another implementation, the power source 3 may be a wired power supply. The wired power supply is directly connected to mains supply through a plug to supply power to the aerosol generating apparatus.

In a preferred embodiment, a direct current supply voltage provided by the battery core is in a range of about 2.5 V to about 9.0 V, and an amperage of the direct current that the battery core may provide is in a range of about 2.5 A to about 20 A.

Power may be supplied to the heater 2 as a pulse signal. An amount of power transmitted to the heater 2 may be adjusted by changing a duty cycle, or a pulse width, or a pulse amplitude of the power signal.

Further, in an optional implementation, the aerosol generating product 1 is preferably made of a tobacco-containing material that releases a volatile compound from a smokable product when being heated, or may be a non-tobacco material suitable for electric heating to smoke after being heated. The aerosol generating product 1 is preferably made of a solid substrate, which may include one or more of powder, particles, fragments, strips, or sheets of one or more of a vanilla leaf, a tobacco leaf, homogeneous tobacco, and expanded tobacco. Alternatively, the aerosol generating product 1 may include an additional tobacco or non-tobacco volatile aroma compound to be released when the aerosol generating product 1 is heated. In some optional implementations, the aerosol generating product 1 is manufactured to be shaped like a conventional cigarette or cigar.

Further, in an optional implementation, the aerosol generating product 1 may be included in a smoking article. During operation, the smoking article including the aerosol generating product 1 may be completely included within the aerosol generating apparatus. In this case, a user may puff on a mouthpiece of the aerosol generating apparatus. The mouthpiece may be any part of the aerosol generating apparatus placed in a mouth of the user for direct inhalation of the aerosol generated by the aerosol generating product 1 or the aerosol generating apparatus. The aerosol is conveyed to the mouth of the user through the mouthpiece. Alternatively, during operation, the smoking article including the aerosol generating product 1 may be partially included within the aerosol generating apparatus. In this case, the user may directly puff on a mouthpiece of the smoking article.

In a preferred implementation, the heater 2 includes magnetic induction materials such as stainless steel of grade 430 (SS430), or stainless steel of grade 420 (SS420), or an alloy material including iron and nickel (such as a permalloy), or the like that may generate heat in a varying magnetic field. In this way, the heater 2 may generate heat in the varying magnetic field, then perform self-heating as a result of generation of an eddy current and magnetic hysteresis in the varying magnetic field, and transfer and/or radiate heat to the aerosol generating product 1, to heat the aerosol generating product 1. Correspondingly, the aerosol generating apparatus further includes a magnetic field generator, for example, an induction coil, which is configured to generate a varying magnetic field under an alternating current, and a controller 2 is connected to the battery core and the induction coil, and may convert the direct current outputted by the battery core into an alternating current. Preferably, a frequency of the alternating current is in a range of 80 KHz to 400 KHz. More specifically, the frequency may be approximately in a range of 200 KHz to 300 KHz.

In a preferred implementation, the heater 2 has an infrared coating. The infrared coating receives electric power to generate heat, and then generates an infrared ray of a specific wavelength, for example, a far infrared ray in a range of 8 $\mu$m to 15 $\mu$m. When a wavelength of the infrared ray matches an absorption wavelength of the aerosol generating product 1, energy of the infrared ray is easily absorbed by the aerosol generating product.

The infrared coating is preferably formed by far infrared electrothermal ink, ceramic powder, and an inorganic adhesive through fully and uniformly stirring, then coated on an outer surface of a base body, and then dried and cured for a specified period of time. A thickness of the infrared coating is in a range of 30 $\mu$m to 50 $\mu$m. Certainly, the infrared coating may further be formed by mixing and stirring a specified proportion of stannic chloride, tin oxide, antimony butter, titanium tetrachloride, and anhydrous cupric sulfate, and then coated on the outer surface of the base body. Alternatively, the infrared coating may be one of a silicon carbide ceramic layer, a carbon fiber composite layer, a zirconium-titanium oxide ceramic layer, a zirconium-titanium nitride ceramic layer, a zirconium-titanium boride ceramic layer, a zirconium-titanium carbide ceramic layer, a ferric oxide ceramic layer, a ferric nitride ceramic layer, a ferric boride ceramic layer, a ferric carbide ceramic layer, a rare-earth oxide ceramic layer, a rare-earth nitride ceramic layer, a rare-earth boride ceramic layer, a rare-earth carbide ceramic layer, a nickel-cobalt oxide ceramic layer, a nickel-cobalt nitride ceramic layer, a nickel-cobalt boride ceramic layer, a nickel-cobalt carbide ceramic layer, or a high silica molecular sieve ceramic layer. The infrared coating may alternatively be an existing coating made of another material.

In a preferred implementation, the heater 2 is made of a resistive conductive material such as an iron chromium aluminum alloy, a nickel chromium alloy, a nickel iron alloy, platinum, tungsten, silver, or a conductive ceramic, or a conductive material including at least one of the above, so that heat may be generated through resistance during conduction, to heat the aerosol generating product 1. Therefore, at least one component in the aerosol generating product 1 is volatilized to form an aerosol.

In a preferred implementation, the heater 1 is substantially in a shape of a pin, or a needle, or a token, which facilitates insertion into the aerosol generating product 1. In addition, the heater 2 may have a length of about 12-19 millimeters, a diameter of 2.0-2.6 mm, and a cross section in a shape of a circle, a straight line, an ellipse, a polygon, or the like. In another embodiment, the heater 2 may be substantially cylindrical, or another structure that can be arranged on a periphery of the aerosol generating product 1 to transfer and radiate heat from the periphery of the aerosol generating product 1 to the aerosol generating product.

The aerosol generating apparatus may include a single heater 2. Alternatively, the aerosol generating apparatus may include more than one heater 2. The heater 2 or the plurality of heaters 2 may be appropriately arranged to most effectively heat the aerosol generating product 1. The plurality of heaters 2 may be configured to heat the aerosol generating product in sections, and at least two heaters 2 among the plurality of heaters 2 may have different heating manners or heating efficiency.

The heater 2 may heat the aerosol generating product 1 through conduction. The heater 2 may be at least partially in contact with the aerosol generating product 1 or a carrier of the aerosol generating product 1. Alternatively, the heat from the heater 2 may be transferred to the aerosol generating product 1 through a thermally conductive element.

Alternatively, the heater 2 may heat the aerosol generating product 1 through convection. Alternatively, ambient air may be heated by the heater 2 before passing through the aerosol generating product 1. Alternatively, the heater 2 may heat the aerosol generating product 1 through radiation.

In an embodiment, the power is supplied to the heater 2 until one or more heaters reach a temperature approximately in a range of 250°C to 440°C, so that the aerosol generating product 1 generates the aerosol.

The aerosol generating apparatus is preferably a handheld aerosol generating apparatus.

In addition, the aerosol generating apparatus includes an insertion detector 4 and a user interface 5 (for example, a graphic display or a combination of light emitting diode (LED) indicator lights) for transmitting information about the aerosol generating apparatus to the user.

The insertion detector 4 may detect a presence and a characteristic of the aerosol generating product 1 close to the heater 2 on a heat transfer path, and send a signal indicating the presence of the aerosol generating product 1 to the controller 6. It may be understood that the provision of the insertion detector 4 is optional but not necessary.

The controller 6 controls the user interface 5 to display system information, for example, battery core power, a temperature, a state of the aerosol generating product 1, a number of puffs, other information, or a combination thereof.

The controller 6 is electrically connected to the power source 3 and the heater 2, and is configured to control an output of a current, a voltage, or electrical power of the power source 3, and detect a temperature change speed of an actual temperature of the heater 2 relative to a target temperature, or the like. Certainly, it is not excluded that in another embodiment, the controller 6 may further obtain the temperature change speed of the actual temperature of the heater 2 relative to the target temperature or the like in a manner other than through detecting the actual temperature of the heater 2, for example, through detecting resistance or a resistance change of the heater.

The controller 6 may include a programmable microprocessor. In another embodiment, the controller 6 may include a dedicated electronic chip, for example, a field-programmable gate array (FPGA) or an application-specific integrated circuit (ASIC). Generally, any apparatus that can provide a signal capable of controlling the heater may be used together with the embodiments discussed herein. In an embodiment, the controller 6 is constructed to detect the temperature change speed of the actual temperature of the heater 2 relative to the target temperature, to detect a user puffing event.

The controller 6 may include a storage component. The storage component may include a memory and/or a cache. The storage component may be constructed to record a detected change in an airflow or the user puff. The storage component may record a number of puffs of the user or time of each puff. The storage component may be further constructed to record a temperature of a heater element and power supplied during each puff. The recorded data may be displayed through the user interface 5 under invoking of the controller 5, or outputted and displayed through another output interface. When the recorded number of puffs reaches a preset total number of puffs for the aerosol generating product, the controller 6 may be reset, or the controller 2 may clear the recorded number of puffs, or the controller 6 may control the aerosol generating apparatus to shut down, or the controller 6 may control the power source 3 to stop supplying power to the heater 2, or the controller may prompt the user through a sound, light, vibration, or the like that the aerosol generating product 1 has reached a puffing limit.

The user puffing may be useful for subsequent research and apparatus maintenance and design. Data of the number of puffs of the user may be transmitted to an external memory or processing apparatus through any appropriate data output apparatus. For example, the aerosol generating apparatus may include radio and Bluetooth connected to the controller 6 or the memory, or a universal serial bus (USB) slot connected to the controller 6 or the memory. Alternatively, the aerosol generating apparatus may be constructed to transfer data from the memory to an external memory in a battery core charging apparatus each time the aerosol generating apparatus is recharged through appropriate data connection.

The actual temperature of the heater 2 is affected by power supplied to the heater, and the heater 2 is cooled by an airflow passing through the heater 2 during the puffing, to cool a temperature of the heater. The cooling effect may be used for detecting the user puffing event. When the airflow increases as a result of the puffing, the actual temperature of the heater 2 is to decrease.

This application provides an apparatus for detecting a change in an airflow flowing through the aerosol generating apparatus, particularly detecting inhalation or puffing of the user without a need for a dedicated airflow sensor. Compared to an existing apparatus that includes the dedicated airflow sensor, this application has reduced costs and complexity of detecting user inhalations, and has increased reliability as a result of a presence of a reduced number of components that may possibly fail.

The aerosol generating apparatus usually requires high power for an initial period of time at the beginning of usage, so that the heater 2 rises to a first target temperature as quickly as possible for the user to take a first puff. Once the first target temperature is reached, the applied power decreases to maintain the heater 2 at a second target temperature, to meet a subsequent puffing requirement of the user.

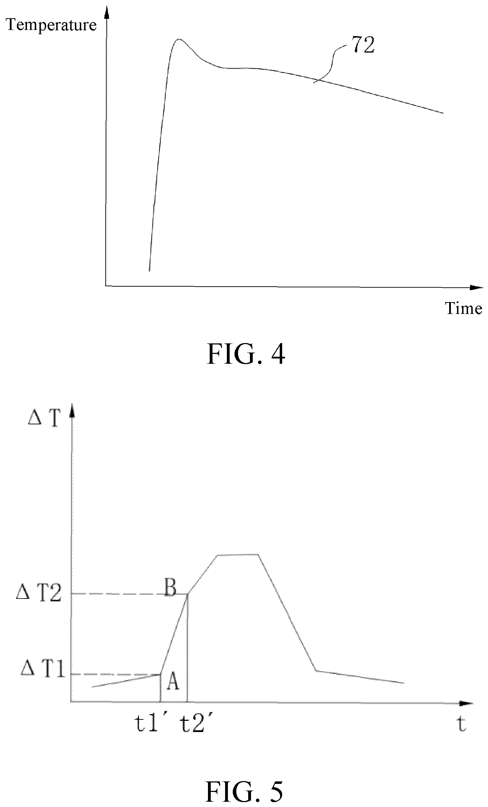

However, in an actual application, after the aerosol generating product 1 is placed in the aerosol generating apparatus, the power provided by the power source 3 to the heater 2 is not a constant value. Referring to FIG. 3 and FIG. 4, FIG. 3 is a graph of an ideal state of a target temperature of a heater during user puffing. A line 71 represents the target temperature in the ideal state, which is a reference temperature. At the reference temperature, the above second target temperature is almost constant. The constant temperature described in this application includes a very small temperature change. For example, when a temperature change does not exceed an error temperature within a temperature detection period, it is considered that the temperature is a constant temperature. The error temperature may be ± 5°C, or the like. Due to a factor such as line damage, interference, a reduction in substrates in the aerosol generating product, or energy saving, FIG. 4 is a graph of an actual state of a target temperature of a heater 2 during user puffing. A line 72 represents the target temperature in the actual state. Therefore, in practice, the target temperature varies over time and may even fluctuate. The target temperature corresponding to each puff of a user may not be the same. In some embodiments, a target temperature of an $(N+M)^{th}$ puff of the user is less than a target temperature of an $N^{th}$ puff of the user, where N is an integer greater than 1, and M is an integer greater than 0. In some embodiments, as a number of puffs of the user increases, the target temperature gradually decreases. Power provided by a power source 3 to the heater 2 is not constant. Usually, after puffing for a period of time, the power provided by the power source 3 to the heater 2 gradually decreases, which also leads to a decrease in the target temperature to some extent.

An airflow inhaled by the user flowing through the heater 2 takes away heat of the heater 2, so that a temperature of the heater 2 decreases during the puffing. However, when the power provided by the power source 3 to the heater 2 decreases, or another reason causes a fluctuation event of the target temperature after a first puff, it is not accurate to only check a difference between a real-time temperature of the heater 2 and a default constant target temperature to represent the user puffing event. For example, an increase or a decrease in the difference between the real-time temperature of the heater 2 and the target temperature may be caused by a decrease or an increase in the target temperature, rather than a change in the actual temperature of the heater 2, resulting in erroneous detection or erroneous determining. To overcome the problem and eliminate the interference of the change in the target temperature on a detection result, in an embodiment of this application, a controller is adopted to detect the actual temperature of the heater 2, determine a temperature change speed of an actual temperature of the heater 2 relative to the target temperature, and count a number of puffs of the user when the temperature change speed meets a preset condition. Certainly, it may be understood that the technical solutions such as determining the temperature change speed of the actual temperature of the heater 2 relative to the target temperature adopted in this application may also be applied to a case in which the target temperature remains constant after the first puff, which is used as a basis for counting the number of puffs of the user.

In an embodiment, the temperature change speed of the actual temperature of the heater 2 relative to the target temperature may be a change amount of the actual temperature of the heater 2 relative to the target temperature per unit time, that is, $[(T_{actual\ 1}-T_{actual\ 2})-(T_{target\ 1}-T_{target\ 2})]/\Delta t$. $T_{target\ 2}$ is a target temperature at a moment $t_2$, $T_{target\ 1}$ is a target temperature at a moment $t_1$, $T_{actual\ 2}$ is an actual temperature of the heater 2 at the moment $t_2$, $T_{actual\ 1}$ is an actual temperature of the heater 2 at the moment $t_1$, $\Delta t$ is a unit time, and $\Delta t=t_2-t_1$. In some embodiments, the temperature change speed of the actual temperature of the heater 2 relative to the target temperature may be a first derivative obtained through calculation of a time derivative of a difference between the target temperature and the temperature of the heater 2.

The temperature change speed of the actual temperature of the heater 2 relative to the target temperature is confirmed through a controller 6, which may eliminate impact of the change in the target temperature on the detection result, thereby avoiding false detection or erroneous determining of counting the number of puffs of the user. If the target temperature is not changed, $T_{target\ 1}-T_{target\ 2}=0$, which does not affect the detection result. Therefore, regardless of whether the target temperature changes, the solution provided in this application may be adopted to count the number of puffs of the user.

To avoid an interference event, for example, interference with a counting result caused by a temporary fluctuation or the like on the temperature of the heater 2 as a result of a power supply fluctuation due to a small airflow fluctuation, magnetic interference, or the like, the controller 6 is preferably constructed to detect whether the temperature change speed of the actual temperature of the heater 2 relative to the target temperature exceeds a first threshold. If the temperature change speed is less than or equal to the first threshold, it is considered that an interference event occurs, and the number of puffs of the user is not counted, thereby improving accuracy of counting the number of puffs of the user. If the temperature change speed is greater than the first threshold, it is determined that a user puffing time occurs, or a next step may be performed for more accurate determining. When it is determined that a user puffing event occurs, the controller controls to add 1 to the number of puffs of the user each time, to count the number of puffs of the user. In other words, in some embodiments, the temperature change speed being greater than the first threshold becomes a preset condition for the controller 6 to detect the user puffing event.

To further provide accuracy of detecting the user puffing event, in a further embodiment, the controller 6 confirms an acceleration of the temperature change speed of the actual temperature of the heater 2 relative to the target temperature. In an embodiment, the acceleration is a change rate of the above temperature change speed per unit time, which is used for representing a change trend of the temperature change speed of the actual temperature of the heater relative to the target temperature. Alternatively, the acceleration may be a time derivative of the temperature change speed of the actual temperature of the heater 2 relative to the target temperature, or a second derivative obtained through calculation of a second time derivative of the change amount of the actual temperature of the heater 2 relative to the target temperature. When the acceleration is greater than 0, it represents that the temperature change speed is increasing, and represents that an absolute value of the difference between the actual temperature of the heater 2 and the target temperature is continuously and quickly increasing. When the acceleration decreases to zero, it represents that the temperature change speed of the actual temperature of the heater 2 relative to the target temperature reaches a maximum. In this case, it represents that a speed or a flow rate of an airflow flowing through the heater 2 reaches a maximum.

In a preferred embodiment, the controller 6 is constructed to determine whether the acceleration of the temperature change speed of the actual temperature of the heater 2 relative to the target temperature is greater than a second threshold. If the acceleration is not greater than the second threshold, it may be considered that the change in the temperature on the heater 2 is caused by a non-puffing event, to eliminate interference of an event such as accelerated heat dissipation and temperature reduction of the heater 2 as a result of a sudden change in an ambient temperature. For example, when the user suddenly enters a low-temperature indoor environment from a high-temperature outdoor environment without puffing, the ambient temperature suddenly changes, which is likely to cause the accelerated heat dissipation of the heater 2, resulting in a decrease in the temperature, thus constituting an interference event for determining the user puffing event. The acceleration of the temperature reduction of the heater 2 caused by the interference event is usually less than the acceleration of the temperature reduction of the heater 2 caused by the puffing event. Therefore, the acceleration being greater than the second threshold is used as a determining condition, which may further reduce erroneous determining events and improve counting accuracy. In other words, in some embodiments, the acceleration being greater than the second threshold becomes a preset condition for the controller 6 to detect the user puffing event.

In a preferred embodiment, the controller 2 is constructed to adjust the power supplied to the heater 2 when it is detected that the acceleration drops to the second threshold. For example, when detecting that the acceleration drops to zero (that is, the second threshold is zero), the controller 6 controls the power source 3 to increase the power supplied to the heater 2, to compensate for a temperature loss on the heater 2, and quickly adjust the temperature back to the corresponding target temperature. Certainly, this is not limited thereto. To be specific, the controller 6 is constructed to adjust the power supplied to the heater 2 at a suitable moment it is detected that the temperature of the heater does not match the target temperature at a corresponding moment, to increase or decrease the power supplied to the heater 2, so as to maintain the heater 2 at the corresponding target temperature. After the temperature compensation or the temperature adjustment is performed on the heater 2, the difference between the corresponding target temperature and the actual temperature thereof decreases, the acceleration decreases, and the temperature change speed decreases.

In a preferred embodiment, the controller 6 is constructed to detect an absolute change amount of the actual temperature of the heater 2 relative to the target temperature, to detect the user puffing event. Referring to FIG. 5, when a temperature change speed of an actual temperature of a heater 2 relative to a target temperature is a first threshold, a difference between the target temperature and the actual temperature of the heater 2 is $\Delta T1$, and a corresponding time is 11', for which reference may be made to a point A in a model shown in FIG. 5. When an acceleration of the temperature change speed of the actual temperature of the heater 2 relative to the target temperature is a second threshold, or when the temperature change speed reaches a preset speed, the difference between the target temperature and the actual temperature of the heater 2 is $\Delta T2$, and the corresponding time is t2', for which reference may be made to a point B in the model shown in FIG. 5. The preset speed is equal to the temperature change speed when the acceleration is the second threshold. From t1' to t2', the acceleration gradually decreases. When the acceleration is 0, the temperature change speed reaches a maximum, so that the preset speed is a maximum speed that the temperature change speed can reach. It is not excluded that in another mathematical model, within a temperature detection period, the temperature change speed equal to the first threshold also uniquely corresponds to $\Delta T1$, and the preset speed uniquely corresponds to $\Delta T2$.

An absolute change amount $\Delta = \Delta T2 - \Delta T1$. To enable the absolute change amount $\Delta$ to be more easily identified or captured, preferably, when the acceleration of the temperature change speed of the actual temperature of the heater 2 relative to the target temperature drops to zero, the difference between the target temperature and the actual temperature of the heater 2 is $\Delta T2$.

In a preferred embodiment, a controller 6 is constructed to determine whether the absolute change amount $\Delta$ exceeds a third threshold. If the absolute change amount does not exceed the third threshold, it is determined that a non-puffing event occurs. If the absolute change amount exceeds the third threshold, it is considered that a puffing event occurs, and a number of puffs may be increased by one. In other words, in some embodiments, whether the absolute change amount $\Delta$ exceeds the third threshold becomes a preset condition for the controller 6 to detect the user puffing event.

Due to an electrical disconnection or short circuit event between a power source 3 and the heater 2, or another non-puffing event, a change in the actual temperature on the heater 2 accelerates. For example, the heater 2 is temporarily disconnected, and a power source 3 stops supplying power to the heater 2, so that the heater 2 starts to naturally dissipate heat and cool down. In this case, a lower ambient temperature of the heater indicates a larger natural cooling speed and a larger acceleration. However, in this case, a temperature change trend on the heater 2 is relatively slower than a temperature change trend on the heater caused by the puffing event. Within the same time, an absolute change amount caused by the above non-puffing event is relatively small. Therefore, the third threshold may be used to eliminate interference of such events on counting of the number of puffs, and improve accuracy of counting the number of puffs.

In a preferred embodiment, the controller 6 is constructed to detect an absolute change amount slope of the actual temperature of the heater 2 relative to the target temperature, to detect the user puffing event.

The absolute change amount slope is equal to $\Delta/(t2'-t1')=(\Delta T2-\Delta T1)/(t2'-t1')$. Similarly, if the absolute change amount slope exceeds a fourth threshold, it is considered that the user puffing event is detected, and the controller adds 1 to the number of puffs each time. Otherwise, it is determined that a non-puffing event occurs. In other words, in some embodiments, the absolute change amount slope being greater than the fourth threshold becomes a preset condition for the controller 6 to detect the user puffing event.

To cause the absolute change amount slope to be easily identified, preferably, when the acceleration of the temperature change speed of the actual temperature of the heater 2 relative to the target temperature drops to zero, the difference between the target temperature and the actual temperature of the heater 2 is $\Delta T2$.

As the number of puffs increases, the power supplied by the power source 3 to the heater 2 gradually decreases, and the target temperature also gradually decreases. To avoid missing detection of the number of puffs, in a preferred embodiment, the controller 2 is constructed to reduce at least one of the first threshold, the third threshold, and the fourth threshold after addition of 1 to the number of puffs each time, and preferably reduce the first threshold and the third threshold appropriately. A reduction amount thereof is related to hardware configuration of a specific aerosol generating apparatus or component characteristics of an aerosol generating product. In an embodiment, a first threshold of an $(N+M)^{th}$ puff of the user is less than a first threshold of an $N^{th}$ puff of the user, where N is an integer greater than or equal to 2, and M is an integer greater than or equal to 1; and/or a third threshold of the $(N+M)^{th}$ puff of the user is less than a third threshold of the $N^{th}$ puff of the user, N is an integer greater than or equal to 2, and M is an integer greater than or equal to 1. It may be understood that reducing the fourth threshold has the same effect as reducing the third threshold.

In another embodiment, the temperature change speed of the actual temperature of the heater 2 relative to the target temperature may be a ratio of a change amount of the actual temperature of the heater 2 per unit time relative to a change amount of the target temperature per unit time, that is, $(T_{actual\ 2}-T_{actual\ 1})/(T_{target\ 2}-T_{target\ 1})$. $T_{target\ 2}$ is a target temperature at a moment $t_2$, $T_{target\ 1}$ is a target temperature at a moment $t_1$, $T_{actual\ 2}$ is an actual temperature of the heater 2 at the moment $t_2$, and $T_{actual\ 1}$ is an actual temperature of the heater 2 at the moment $t_1$. For example, when $(T_{actual\ 2}-T_{actual\ 1})/(T_{target\ 2}-T_{target\ 1})$ exceeds a threshold, the controller 6 may determine that the user puffing event occurs, and then add 1 to the number of puffs each time. In other words, $(T_{actual\ 2}-T_{actual\ 1})/(T_{target\ 2}-T_{target\ i})$ exceeding the threshold becomes a preset condition for the controller 6 to detect the user puffing event.

In an embodiment, as shown in FIG. 2, the actual temperature of the heater 2 may be detected through a dedicated temperature sensor 8. The temperature sensor 8 is connected to the controller 6 and the heater 2, and is configured to obtain the temperature of the heater 2, and then transmit the temperature to the controller 6. Referring to FIG. 2, the controller 6 forms two closed loops with the power source 3 and the heater 2. First closed loop: The controller 6 is connected to a first switch K1 through a first pin of the controller for outputting a first pulse signal PWM1, and adjusts the power supplied to the heater 2 through the first switch K1 in cooperation with a boost inductor W, and then the boost inductor W is connected to a first conversion pin ADC1 of the controller to form a feedback loop. Second closed loop: The controller 6 is connected to a second switch K2 through a second pin of the controller for outputting a second pulse signal PWM2, and is configured to start the heater 2 through the second switch K2, and then the temperature sensor 8 is connected to the heater 2 and a second conversion pin ADC1 of the controller 6 to form a feedback loop. To prevent an accidental power failure of the heater, the heater 2 may be connected in parallel with a capacitor C.

To ensure puffing experience of the user, when the controller 6 detects that the temperature of the heater does not match the target temperature or detects the puffing event, the controller 6 may control the first switch K1, and use the boost inductor W to increase the power supplied to the heater 2. Certainly, the power supplied to the heater 2 may further be increased by changing a duty cycle of a power signal or the like, or the power supplied to the heater 2 may be increased in another manner, to ensure that aerosol generating product 1 can be heated and quickly generate sufficient aerosols for the user to inhale during user puffing.

In another embodiment, the controller 6 may be constructed to detect the actual temperature of the heater 2 based on a measured value of resistance of the heater 2. This enables the actual temperature of the heater 2 to be detected without a need for additional sensing hardware.

Specifically, a resistivity of a conductor depends on a temperature thereof. A resistivity $\rho$ varies with the temperature. A characteristic of the actual resistivity $\rho$ varies based on an exact component of the conductor and a geometric configuration of the heater 2, and an empirically determined relationship may be used in the controller 6. Therefore, a resistivity $\rho$ obtained at any given time may be used to derive the actual temperature of the heater 2 of the heater element.

A resistance R of the heater 2 is equal to V/I, where V is a voltage across the heater element, and I is a current passing through the heater. The resistance R depends on the configuration and the temperature of the heater, and is expressed through the following relation:

$$R=\rho(T)*L/S\ (1),$$

where $\rho(T)$ is a temperature-dependent resistivity, L is a length, and S is a sectional area of the heater 2. For the given configuration of the heater 2, L and S are fixed and measurable. Therefore, for a design of the given heater 2, R is directly proportional to $\rho(T)$.

The resistivity $\rho$ (T) of the heater 2 may be expressed by the following polynomial:

$$\rho(T)=\rho_0*(1+\alpha_1 T+\alpha_2 T^2) \ (2),$$

where $\rho_0$ is a resistivity at a target temperature T0, and $\alpha_1$ and $\alpha$ are polynomial coefficients.

Therefore, a length and a section of the heater 2 are learned. Through measurement of a voltage V and a current I of the heater 2, a resistance R at a given temperature can be determined, and therefore a resistivity $\rho$ can be determined. The temperature may be simply obtained through a lookup table of a relationship between the resistivity characteristic of the used heater 2 and the temperature, or through solution of the polynomial of the above equation (2). In an embodiment, a curve of the resistivity $\rho$ relative to the temperature is represented by using one or more (preferably two) linear approximations within a temperature range applied to the aerosol generating product 1, which may simplify processing. This simplifies estimation of the temperature, which is desirable in the controller 6 having limited computing resources.

The controller 6 includes a measurement unit and a control unit. The measurement unit is constructed to determine the resistance R of the heater 2. The measurement unit transmits a resistance measurement value to the control unit. Then the control unit controls power supplied by a battery core to the heater 2 by switching a switch. The controller 6 may include a microprocessor and a separate electronic control circuit. In an embodiment, in addition to another function, the microprocessor may include a standard function, for example, an internal clock.

In a preparation step for temperature control, a value of the target temperature for the aerosol generating apparatus is selected. The selection is based on a release temperature of a volatile compound that should or should not be released. Then the value of the target temperature is stored in the control unit. The control unit includes a non-volatile memory.

By controlling electrical energy supplied by the battery core to the heater 2, the controller 6 controls heating of the heater 2. If the insertion detector 4 has detected the aerosol generating product 1 and the user has started the apparatus, the controller 6 allows the power to be supplied only to the heater 2. Through switching of a switch K1, the power is provided as a pulse signal. A pulse width or a duty cycle of the signal may be modulated by the control unit to change energy supplied to the heater 2. In an embodiment, the duty cycle may be limited to a range of 60% to 80%. This may provide additional safety and prevent the user from inadvertently raising a compensation temperature of the heater 2, to cause the aerosol generating product 1 to reach a temperature above an aerosol generating temperature.

In use, the controller measures the resistivity $\rho$ of the heater 2. The controller 6 then converts the resistivity of the heater into a value of the actual temperature of the heater by comparing the measured resistivity $\rho$ with the lookup table. This may be completed within the measurement unit or through the control unit. In the following steps, the controller 6 compares an actually exported operating temperature with the target temperature. Alternatively, a temperature value in a heating curve is converted into a resistance value in advance, so that the controller 6 adjusts the resistance instead of adjusting the temperature, which avoids real-time calculation for converting the resistance into the temperature during smoking experience.

If the actual temperature of the heater 2 is less than the target temperature, the control unit supplies additional electrical energy to the heater 2 or boosts the voltage of the heater 2 through the boost inductor W, so as to increase the actual temperature of the heater 2. If the actual temperature of the heater 2 is higher than the target temperature, the control unit reduces the electrical energy supplied to the heater 2, so as to reduce the operating temperature back to the target temperature.

The control unit may implement any appropriate control technology to adjust the temperature, for example, a simple constant temperature feedback loop or a proportional integral derivative (PID) control technology.

In use of the PID control technology, assuming that e(t)=0 before the puffing, a control rule is controlled as follows:

$$e(t) = \frac{P_N}{c*b}\left[e^{-at}\sin(bt)u(t) - e^{-a(t-\alpha)}\sin\big(b(t-\alpha)\big)*u(t-\alpha)\right]$$

$$a = \frac{K_p}{2c}, \text{b} = \sqrt{\frac{K_i}{c} - \left(\frac{K_p}{2c}\right)^2}$$

where $P_N$ is power lost during the puffing, $\alpha$ is a puffing duration, c is a specific heat capacity, u(t) is a unit step function, $K_p$ is a proportional coefficient, and $K_i$ is an integral coefficient. The equation indicates that the puffing causes a gradually decaying oscillation. A larger $K_p$ leads to faster decay. However, an excessively large $K_p$ may cause the controller 6 to easily lose stability. A smaller $K_i$ leads to a lager oscillation period of the controller 6. However, an excessively small $K_i$ increases a steady-state error. For convenience of counting of the number of puffs, $K_p$ and $K_i$ may be adjusted such that only one oscillation occurs.

In an embodiment, the actual temperature of the heater 2 may be detected at a predetermined time interval, for example, every few milliseconds, which may be implemented continuously or only during a period when the power is supplied to the heater 2.

The aerosol generating product 1 has a preset total number of puffs. The controller 6 may be constructed to reset when a detected number of puffs exceeds the preset total number of puffs, and prepare to detect that the user puffs a next aerosol generating product. When a total number of puffs counted by the controller 6 is equal to the preset total number of puffs, it may be considered that the aerosol generating product 1 has been completely inhaled, and the aerosol generating product 1 may be replaced. If the aerosol generating apparatus is a disposable apparatus, it represents that the aerosol generating apparatus may be replaced. When the total number of puffs counted by the controller 6 is equal to the preset total number of puffs, prompt information such as a sound, a vibration, or light may be transmitted, to prevent a reduction in user experience as a result of the user continuously inhaling without knowing the situation.

In an embodiment, the controller 6 further includes a signal processor. The actual temperature information on the heater 2 obtained by the measurement unit passes through the signal processor and then is transmitted to the control unit in the controller 6, and then the control unit compares and calculates the actual temperature of the heater 2 with the target temperature. On one hand, the signal processor may perform noise reduction on the temperature information, to eliminate interference from an airflow other than an airflow generated by non-puffing and/or eliminate electrical interference, magnetic interference, or the like. On the other hand, the signal processor may convert the collected temperature information into a signal that can be recognized by the control unit. After the noise reduction performed by the signal processor, the measured temperature of the heater 2 may be lower than the target temperature.

Specifically, the signal processor may be a digital filter. More specifically, the signal processor may be a low-pass filter having a cut-off frequency of 0.5 Hz. Only a signal with a period of more than 2 seconds is retained. In an actual application, an appropriate filter may be selected based on a specific application need.

In an embodiment, a method for counting a number of puffs is provided. The method is based on a temperature change speed of a temperature of a heater 2 relative to a target temperature.

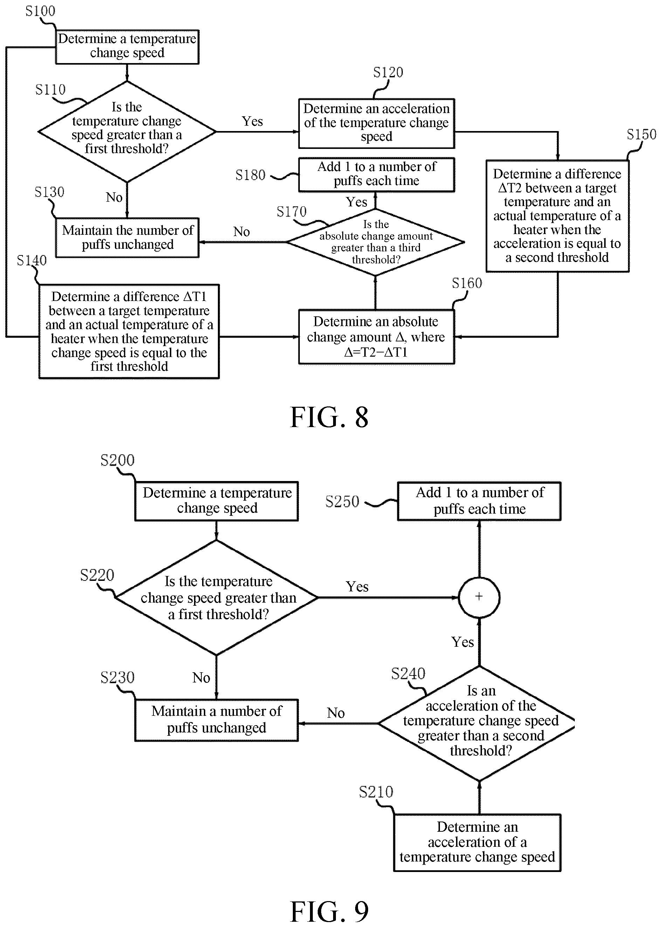

Refer to FIG. 8. S100: Obtain a temperature change speed of an actual temperature of a heater 2 relative to a target temperature. Step S110: Determine whether the speed is greater than a first threshold. If the speed is greater than the first threshold, step S120 of starting to detect an acceleration of the temperature change speed of the actual temperature of the heater 2 relative to the target temperature is performed. If the speed is not greater than the first threshold, step S130 of determining that an aerosol generating apparatus is in a non-puffing state and maintaining a number of puffs unchanged is performed. Step S140: Determine that a corresponding time is t1' and a difference between the target temperature and the actual temperature of the heater is $\Delta T1$ when the temperature change speed of the actual temperature of the heater relative to the target temperature is equal to the first threshold. Step S150: Determine that a corresponding time is $t_2$, and a difference between the target temperature and the actual temperature of the heater is $\Delta T2$ when the acceleration is equal to a second threshold. Step S160: Determine an absolute change amount of the actual temperature of the heater relative to the target temperature, where the absolute change amount $\Delta = T2 - T1$. Step S170: Determine whether the absolute change amount is greater than a third threshold. If the absolute change amount is greater than the third threshold, step S180 of adding 1 to the number of puffs each time is performed. Otherwise, step S130 is performed.

In another embodiment, the above step S160 may alternatively be changed to determining an absolute change amount slope of the actual temperature of the heater relative to the target temperature, where the absolute change amount slope is equal to $\Delta/(t2'-t1')$. Correspondingly, step S170 is changed to determining whether the absolute change amount slope is greater than a fourth threshold. If the absolute change amount slope is greater than the fourth threshold, 1 is added to the number of puffs each time. Otherwise, step S130 is performed.

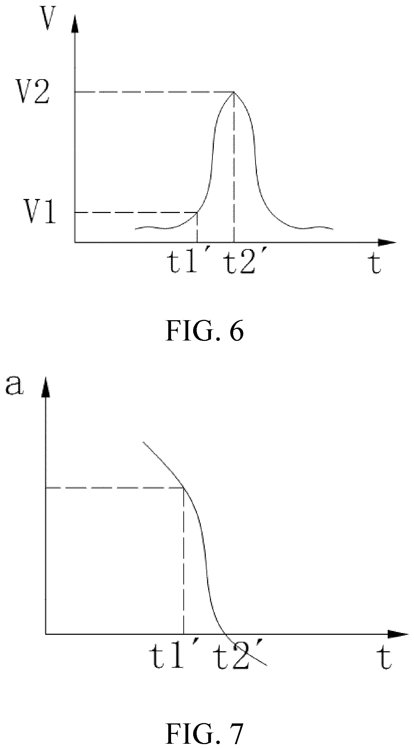

A preset speed is equal to the temperature change speed when the acceleration is the second threshold. During a period from t1' to t2', the acceleration gradually decreases (as shown in a model shown in FIG. 7, in the model, a is the acceleration). When the acceleration is 0, the temperature change speed reaches a maximum, so that the preset speed is a maximum speed that the temperature change speed can reach (as shown in a model shown in FIG. 6, V1 represents the temperature change speed that is equal to the first threshold, and V2 represents the preset speed).

In an embodiment, a method for counting a number of puffs is provided. The method is based on a temperature change speed of a temperature of a heater 2 relative to a target temperature and an acceleration of the temperature change speed.

Refer to FIG. 9. S200: Obtain a temperature change speed of an actual temperature of a heater 2 relative to a target temperature. S210: Obtain an acceleration of the temperature change speed of the actual temperature of the heater 2 relative to the target temperature. Step S220: Determine whether the temperature change speed is greater than a first threshold. If the speed is not greater than the first threshold, step S230 of determining that an aerosol generating apparatus is in a non-puffing state and maintaining a number of puffs unchanged is performed. Step S240: Determine whether the acceleration is greater than a second threshold. If the acceleration is not greater than the second threshold, step S230 of determining that an aerosol generating apparatus is in a non-puffing state and maintaining a number of puffs unchanged is performed. If the speed is greater than the first threshold, and the acceleration is greater than the second threshold, step S250 of adding 1 to the number of puffs each time is performed.

As shown in FIG. 6 and FIG. 7, the temperature change speed and the acceleration are simultaneously confirmed, to avoid erroneous addition of the number of puffs as a result of erroneous determining of an event in which the temperature change speed is greater than the first threshold but the acceleration is less than the second threshold (or the acceleration is negative) as being caused by a puffing event after t2'.

In an embodiment, a method for counting a number of puffs is provided. The method is based on a temperature change speed of a temperature of a heater 2 relative to a target temperature and an acceleration of the temperature change speed.

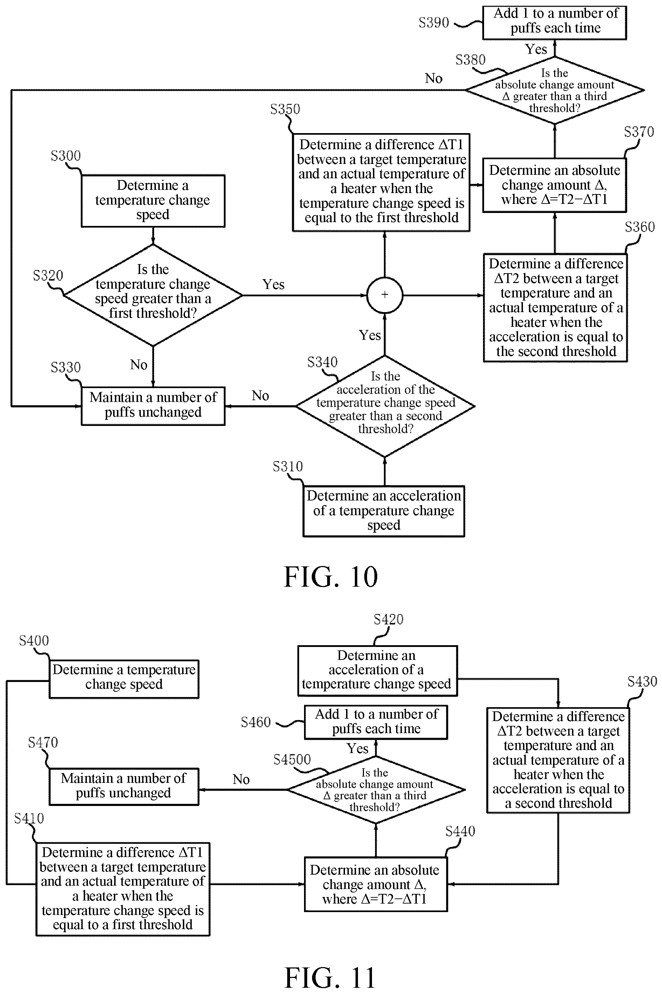

Refer to FIG. 10. S300: Obtain a temperature change speed of an actual temperature of a heater 2 relative to a target temperature. S310: Obtain an acceleration of the temperature change speed of the actual temperature of the heater 2 relative to the target temperature. Step S320: Determine whether the temperature change speed is greater than a first threshold. If the speed is not greater than the first threshold, step S330 of determining that an aerosol generating apparatus is in a non-puffing state and maintaining a number of puffs unchanged is performed. Step S340: Determine whether the acceleration is greater than a second threshold. If the acceleration is not greater than the second threshold, step S330 of determining that an aerosol generating apparatus is in a non-puffing state and maintaining a number of puffs unchanged is performed. If the speed is greater than the first threshold, and the acceleration is greater than the second threshold, step S350 of determining a difference $\Delta T1$ between the target temperature and the actual temperature of the heater 2 when the temperature change speed is equal to the first threshold is performed. Step S360 of determining a difference $\Delta T2$ between the target temperature and the actual temperature of the heater 2 when the acceleration is equal to the second threshold is performed. Step S370: Determine an absolute change amount of the actual temperature of the heater relative to the target temperature, where the absolute change amount $\Delta=T2-T1$. Step S380: Determine whether the absolute change amount is greater than a third threshold. If the absolute change amount is greater than the third threshold, step S390 of adding 1 to the number of puffs each time is performed. Otherwise, step S330 is performed.

In another embodiment, the above step S370 may alternatively be changed to determining an absolute change amount slope of the actual temperature of the heater relative to the target temperature, where the absolute change amount slope is equal to $\Delta/(t2'-t1')$. Correspondingly, step S380 is changed to determining whether the absolute change amount slope is greater than a fourth threshold. If the absolute change amount slope is greater than the fourth threshold, 1 is added to the number of puffs each time. Otherwise, step S330 is performed.

When the temperature change speed is equal to the first threshold, a time is t1'. When the acceleration is the temperature change speed that is equal to the second threshold, and the acceleration is equal to the second threshold, the time is t2'.

In an embodiment, a method for counting a number of puffs is provided. The method is based on a temperature change speed of a temperature of a heater 2 relative to a target temperature and an acceleration of the temperature change speed.

Refer to FIG. 11. Step S400: Determine a temperature change speed. Step S410: Determine a difference $\Delta T1$ between a target temperature and an actual temperature of a heater 2 when the temperature change speed is equal to a first threshold. Step S430: Determine an acceleration of the temperature change speed. Step S440: Determine a difference $\Delta T2$ between the target temperature and the actual temperature of the heater 2 when the acceleration is equal to a second threshold. Step S450: Determine an absolute change amount of the actual temperature of the heater relative to the target temperature, where the absolute change amount $\Delta=T2-T1$. Step S460: Determine whether the absolute change amount is greater than a third threshold. If the absolute change amount is greater than the third threshold, step S470 of adding 1 to the number of puffs each time is performed. Otherwise, step S480 is performed.

In another embodiment, the above step S450 may alternatively be changed to determining an absolute change amount slope of the actual temperature of the heater 2 relative to the target temperature, where the absolute change amount slope is equal to $\Delta/(t2'-t1')$. Correspondingly, step S460 is changed to determining whether the absolute change amount slope is greater than a fourth threshold. If the absolute change amount slope is greater than the fourth threshold, 1 is added to the number of puffs each time. Otherwise, step S480 is performed.

When the temperature change speed is equal to the first threshold, a time is t1'. When the acceleration is the temperature change speed that is equal to the second threshold, and the acceleration is equal to the second threshold, the time is t2'.

A method for counting a number of puffs is provided. The method is based on a temperature change speed of a temperature of a heater 2 relative to a target temperature.

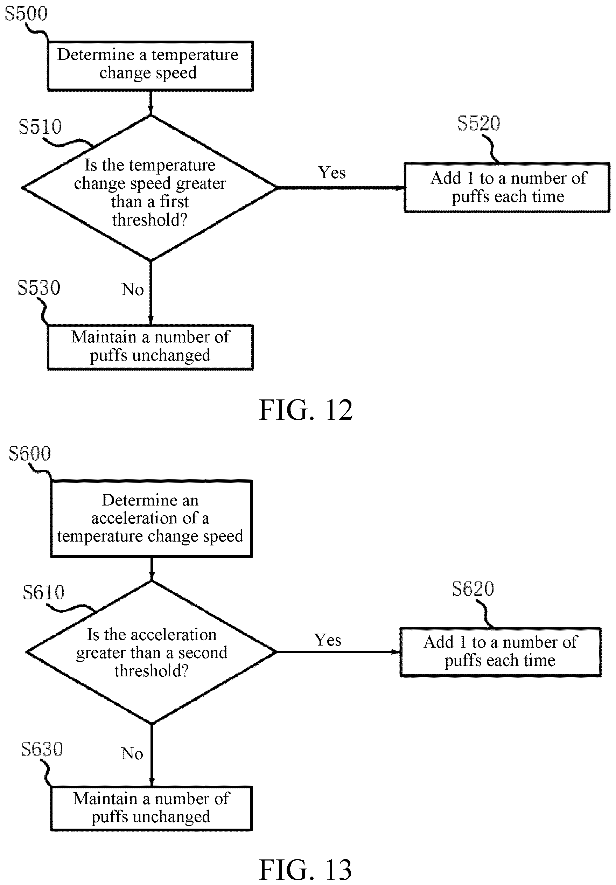

Refer to FIG. 12. Step S500: Determine a temperature change speed. Step S510: Determine whether the temperature change speed is greater than a first threshold. If the temperature change speed is greater than the first threshold, step S520 of adding 1 to a number of puffs each time is performed. Otherwise, step S530 of maintaining the number of puffs unchanged is performed.

To ensure accuracy, time of each detection period, or waiting time for temperature re-sampling after the number of puffs is counted once, or the like needs to be strictly controlled.

In an embodiment, a method for counting a number of puffs is provided. The method is based on an acceleration of a temperature change speed of a temperature of a heater 2 relative to a target temperature.

Refer to FIG. 13. Step S600: Determine an acceleration of a temperature change speed. Step S610: Determine whether the acceleration is greater than a second threshold. If the acceleration is greater than the second threshold, step S620 of adding 1 to a number of puffs each time is performed. Otherwise, step S630 of maintaining the number of puffs unchanged is performed.

To ensure accuracy, time of each detection period, or waiting time for temperature re-sampling after the number of puffs is counted once, or the like needs to be strictly controlled.

This application provides an operating method of an aerosol generating apparatus. The method includes the following steps.

S700: Control power supplied by a power source 3 to a heater 2, to maintain a temperature on the heater 2 at or close to a target temperature. To meet a requirement for quick smoking, referring to FIG. 4, after the aerosol generating product is placed in the aerosol generating apparatus, and the aerosol generating apparatus is started, the power source 3 supplies relatively great power to the heater 2, to cause the heater to quickly rise to the target temperature, so as to heat the aerosol generating product, thereby quickly generating an aerosol.

S710: Detect an actual temperature of the heater 2. A frequency for temperature collection of a controller 6 may be set, to obtain the actual temperature of the heater 2 at a different time in a relatively dense manner.

S720: Determine a temperature change speed of the actual temperature of the heater relative to the target temperature. The controller 6 compares the actual temperature of the heater 2 with the target temperature, and determines a user puffing event based on a change amount of the actual temperature of the heater 2 relative to the target temperature per unit time, that is, the temperature change speed. Compared to simple analysis of the difference between the actual temperature of the heater 2 and the target temperature, the method may eliminate impact of a change in the target temperature on a detection result, thereby reducing erroneous determining, and improving accuracy of detection.

S730: Adjust the power supplied by the power source 3 to the heater 2 when a user puffing event is detected, to cause the temperature of the heater 2 to approach or reach the target temperature, to fully bake the aerosol generating product, so as to meet a puffing requirement of a user.

S740: A controller 6 adds 1 to the number of puffs each time a user puffing event is detected. When the accumulated number of puffs reaches a preset number of puffs of the aerosol generating product, the controller 6 controls the aerosol generating apparatus to transmit a signal such as a sound, light, or a vibration to prompt the user. The power source 3 may be caused to stop supplying the power to the heater 2, or the aerosol generating apparatus may be reset, or the number of puffs may be cleared.

According to the aerosol generating apparatus and the method described above, the change amount of the actual temperature of the heater relative to the target temperature per unit time is confirmed through the controller, to represent the speed of the change of the temperature of the heater relative to the target temperature, so as to more accurately determine the user puffing event, count the number of puffs of the user, avoid missing detection and false detection, and further avoid the impact of the change of the target temperature on the detection result, thereby reducing erroneous determining of the puffing event, and helping improve the accuracy of the detection.

An embodiment of this application further provides a non-volatile computer-readable storage medium, storing computer-executable instructions. The computer-executable instructions, when executed by a controller, for example, the controller 6 in FIG. 1, may cause the controller to perform the method for counting a number of puffs of a user by an aerosol generating apparatus in any of the above method embodiments, for example, to perform step S100 to step S180 of the method in FIG. 8 described above.

An embodiment of this application provides a computer program product, including a computer program stored in a non-volatile computer-readable storage medium. The computer program includes program instructions. The program instructions, when executed by a controller, cause the controller to perform the method for counting a number of puffs of a user by an aerosol generating apparatus in any of the above method embodiments, for example, to perform step S100 to step S180 of the method in FIG. 8 described above.

Through the description of the above implementations, a person of ordinary skill in the art may clearly understand that the implementations may be implemented by software in combination with a universal hardware platform. Certainly, the implementations may alternatively be implemented by hardware. A person of ordinary skill in the art may understand that all or some of processes of the methods in the above embodiments may be implemented by instructing relevant hardware through a computer program. The program may be stored in a computer-readable storage medium. When the program is executed, the processes of the embodiments of the methods described above may be performed. The storage medium may be a magnetic disk, an optical disc, a read-only memory (ROM), a random access memory (RAM), or the like.

It should be noted that, preferred embodiments of this application are provided in the specification and the accompanying drawings of this application, but are not limited to the embodiments described in the specification. Further, a person of ordinary skill in the art may make improvements or modifications based on the above descriptions, and all of the improvements and modifications shall fall within the protection scope of the appended claims of this application.

**Claims**

1. An aerosol generating apparatus, comprising:

   a heater, configured to heat an aerosol generating product, so as to generate an aerosol for a user to inhale;
   a power source, electrically connected to the heater; and
   a controller, connected to the heater, and configured to control the power source to supply power to the heater, to cause a temperature on the heater reaches a target temperature corresponding to each puff of a user, wherein the controller is further configured to: during operation of the aerosol generating apparatus, detect an actual temperature of the heater, and determine a temperature change speed; determine whether the temperature change speed meets a preset condition; add 1 to a number of puffs of the user each time the temperature change speed meets the preset condition, and record the number of puffs, wherein
   the temperature change speed is a change amount of the actual temperature of the heater relative to the target temperature per unit time.

2. The aerosol generating apparatus according to claim 1, wherein the controller is configured to determine whether the temperature change speed is greater than a first threshold, and add 1 to the number of puffs of the user each time the temperature change speed is greater than the first threshold.

3. The aerosol generating apparatus according to claim 1 or 2, wherein the controller is configured to:

   determine an acceleration of the temperature change speed;
   determine whether the acceleration is greater than a second threshold; and
   add 1 to the number of puffs of the user each time the acceleration is greater than the second threshold, wherein the acceleration is a change amount of the temperature change speed per unit time.

4. The aerosol generating apparatus according to claim 1, wherein the controller is configured to:

   determine a difference $\Delta T1$ between the target temperature and the actual temperature of the heater when the temperature change speed is equal to a first threshold;
   determine a difference $\Delta T2$ between the target temperature and the actual temperature of the heater when an acceleration of the temperature change speed is equal to a second threshold;
   determine an absolute change amount $\Delta$ based on $\Delta T2$ and $\Delta T1$;
   determine whether the absolute change amount $\Delta$ is greater than a third threshold; and
   add 1 to the number of puffs of the user each time the absolute change amount $\Delta$ is greater than the third threshold.

5. The aerosol generating apparatus according to claim 4, wherein the absolute change amount $\Delta = \Delta T2 - \Delta T1$.

6. The aerosol generating apparatus according to claim 2 or 4, wherein:

   a first threshold of an $(N+M)^{th}$ puff of the user is less than a first threshold of an $N^{th}$ puff of the user, N is an integer greater than or equal to 2, and M is an integer greater than or equal to 1; and/or
   a third threshold of the $(N+M)^{th}$ puff of the user is less than a third threshold of the $N^{th}$ puff of the user, N is an integer greater than or equal to 2, and M is an integer greater than or equal to 1.

7. The aerosol generating apparatus according to claim 6, wherein the first threshold and/or the third threshold gradually decreases as the number of puffs of the user increases.

8. The aerosol generating apparatus according to claim 3 or 5, wherein when the acceleration is equal to the second threshold, the temperature change speed reaches a maximum.

9. The aerosol generating apparatus according to claim 4, wherein the controller is connected to the power source, and is configured to control the power source to perform power compensation on the heater when the acceleration is equal to the second threshold, to cause the heater to reach the corresponding target temperature.

10. The aerosol generating apparatus according to claim 1, wherein a target temperature of an $(N+M)^{th}$ puff of the user is less than a target temperature of an $N^{th}$ puff of the user, N is an integer greater than or equal to 2, and M is an integer greater than or equal to 1.

11. The aerosol generating apparatus according to claim 10, wherein the target temperature continuously decreases as the number of puffs of the user increases.

12. The aerosol generating apparatus according to claim 1, wherein the target temperature remains unchanged as the number of puffs of the user increases.

13. A method for counting a number of puffs of a user of an aerosol generating apparatus, wherein the aerosol generating apparatus comprises a heater, a power source configured to supply power to the heater, and a controller, and the method comprises:

detecting an actual temperature of the heater;
determining a temperature change speed of the actual temperature of the heater relative to a target temperature, wherein the target temperature is a preset target temperature corresponding to each puff of the user, and the temperature change speed is a change amount of the actual temperature of the heater relative to the target temperature per unit time;
determining whether the temperature change speed meets a preset condition;
adding 1 to the number of puffs of the user each time the temperature change speed meets the preset condition; and
recording the number of puffs.

14. The method according to claim 13, wherein the determining whether the temperature change speed meets a preset condition, and adding 1 to the number of puffs of the user each time the temperature change speed meets the preset condition comprises:

determining whether the temperature change speed is greater than a first threshold; and
adding 1 to the number of puffs of the user each time the temperature change speed is greater than the first threshold.

15. The method according to claim 13 or 14, further comprising:

determining an acceleration of the temperature change speed, wherein the acceleration is a change amount of the temperature change speed per unit time,
wherein the step of determining whether the temperature change speed meets a preset condition and adding 1 to the number of puffs of the user each time the temperature change speed meets the preset condition comprises:

determining whether the temperature change speed is greater than a second threshold; and
adding 1 to the number of puffs of the user each time the acceleration of the temperature change speed is greater than the second threshold.

16. The method according to claim 13, further comprising:

determining a difference $\Delta T1$ between the target temperature and the actual temperature of the heater when the temperature change speed is a first threshold;
determining an acceleration of the temperature change speed, wherein the acceleration is a change amount of the temperature change speed per unit time;
determining a difference $\Delta T2$ between the target temperature and the actual temperature of the heater when the acceleration is greater than a second threshold; and
determining an absolute change amount $\Delta$ of the actual temperature of the heater relative to the target temperature, wherein the absolute change amount is equal to $\Delta T2-\Delta T1$,
wherein the step of adding 1 to the number of puffs of the user each time the temperature change speed meets the preset condition comprises:

determining whether the absolute change amount is greater than a third threshold; and
adding 1 to the number of puffs of the user each time the absolute change amount is greater than the third threshold.

17. The method according to any one of claims 13 to 16, wherein during puffing of the user, the preset target temperature does not remain unchanged.

FIG. 1

FIG. 2

FIG. 3

FIG. 4

FIG. 5

FIG. 6

FIG. 7

S100
Determine a temperature change speed

S110
Is the temperature change speed greater than a first threshold?

Yes →

S120
Determine an acceleration of the temperature change speed

S150
Determine a difference ΔT2 between a target temperature and an actual temperature of a heater when the acceleration is equal to a second threshold

S130 No
Maintain the number of puffs unchanged

S180
Add 1 to a number of puffs each time

Yes

S170
No ←
Is the absolute change amount greater than a third threshold?

S140
Determine a difference ΔT1 between a target temperature and an actual temperature of a heater when the temperature change speed is equal to the first threshold

S160
Determine an absolute change amount Δ, where Δ=T2−ΔT1

FIG. 8

S200
Determine a temperature change speed

S250
Add 1 to a number of puffs each time

S220
Is the temperature change speed greater than a first threshold?

Yes →

+

S230 No
Maintain a number of puffs unchanged

No ←

Yes

S240
Is an acceleration of the temperature change speed greater than a second threshold?

S210
Determine an acceleration of a temperature change speed

FIG. 9

S390 — Add 1 to a number of puffs each time

Yes

S380 — No

S380 Is the absolute change amount Δ greater than a third threshold?

S350 Determine a difference ΔT1 between a target temperature and an actual temperature of a heater when the temperature change speed is equal to the first threshold

S370 Determine an absolute change amount Δ, where Δ=T2−ΔT1

S300 Determine a temperature change speed

S360 Determine a difference ΔT2 between a target temperature and an actual temperature of a heater when the acceleration is equal to the second threshold

S320 Is the temperature change speed greater than a first threshold?

Yes

+

Yes

S330 No Maintain a number of puffs unchanged

No

S340 Is the acceleration of the temperature change speed greater than a second threshold?

S310 Determine an acceleration of a temperature change speed

## FIG. 10

S420 Determine an acceleration of a temperature change speed

S400 Determine a temperature change speed

S430 Determine a difference ΔT2 between a target temperature and an actual temperature of a heater when the acceleration is equal to a second threshold

S460 — Add 1 to a number of puffs each time

Yes

S470 Maintain a number of puffs unchanged

No

S4500 Is the absolute change amount Δ greater than a third threshold?

S410 Determine a difference ΔT1 between a target temperature and an actual temperature of a heater when the temperature change speed is equal to a first threshold

S440 Determine an absolute change amount Δ, where Δ=T2−ΔT1

## FIG. 11

S500

Determine a temperature change speed

S510

Is the temperature change speed greater than a first threshold? — Yes → S520 — Add 1 to a number of puffs each time

No

S530

Maintain a number of puffs unchanged

FIG. 12

S600

Determine an acceleration of a temperature change speed

S610

Is the acceleration greater than a second threshold? — Yes → S620 — Add 1 to a number of puffs each time

No

S630

Maintain a number of puffs unchanged

FIG. 13

<div align="center">**INTERNATIONAL SEARCH REPORT**</div>

| International application No. |
|---|
| **PCT/CN2023/084846** |

| **A.** | **CLASSIFICATION OF SUBJECT MATTER** |
|---|---|
| | A24F 40/40(2020.01)i |

According to International Patent Classification (IPC) or to both national classification and IPC

| **B.** | **FIELDS SEARCHED** |
|---|---|

Minimum documentation searched (classification system followed by classification symbols)

IPC: A24F

Documentation searched other than minimum documentation to the extent that such documents are included in the fields searched

Electronic data base consulted during the international search (name of data base and, where practicable, search terms used)

CNTXT, ENTXTC, CNKI, DWPI: 合元科技, 樊珑, 龙智明, 余培侠, 徐中立, 李永海, 气溶胶, 烟, 雾化, 加热, 抽, 吸, 温度, 目标, 预设, 阈值, 差, 速率, 加速度, 斜率, 微分, 导数, 计数, 次数, 一次, smoke, atomiz+, heat, temperature, target, preset, threshold, differ+, velocity, accelerat+, slope, derivative, count, number, times

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| Y | CN 110191650 A (KT&G CORP.) 30 August 2019 (2019-08-30)<br>description, paragraphs [0189]-[0193], and figures 3 and 13 | 1-3, 6-8, 10-15, 17 |
| Y | CN 111671166 A (O-NET AUTOMATION TECHNOLOGY (SHENZHEN) LTD.) 18 September 2020 (2020-09-18)<br>description, paragraphs [0092]-[0100] | 1-3, 6-8, 10-15, 17 |
| A | CN 109998178 A (MYSMOK ELECTRONIC TECHNOLOGY CO., LTD.) 12 July 2019 (2019-07-12)<br>entire document | 1-17 |
| A | CN 112471612 A (HUIZHOU PEIGESI TECHNOLOGY CO., LTD.) 12 March 2021 (2021-03-12)<br>entire document | 1-17 |
| A | US 2021015165 A1 (DONGGUAN MYSMOK ELECTRONIC TECHNOLOGY CO., LTD.) 21 January 2021 (2021-01-21)<br>entire document | 1-17 |

| ☑ Further documents are listed in the continuation of Box C. | ☑ See patent family annex. |
|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **11 May 2023** | **19 May 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/084846**

**C.    DOCUMENTS CONSIDERED TO BE RELEVANT**

| Category* | Citation of document, with indication, where appropriate, of the relevant passages | Relevant to claim No. |
|---|---|---|
| A | WO 2021220384 A1 (JAPAN TOBACCO INC.) 04 November 2021 (2021-11-04)<br>entire document | 1-17 |

Form PCT/ISA/210 (second sheet) (July 2022)

**INTERNATIONAL SEARCH REPORT**
Information on patent family members

International application No.

**PCT/CN2023/084846**

| Patent document cited in search report | | | Publication date (day/month/year) | Patent family member(s) | | | Publication date (day/month/year) |
|---|---|---|---|---|---|---|---|
| CN | 110191650 | A | 30 August 2019 | US | 2019380389 | A1 | 19 December 2019 |
| | | | | US | 11583008 | B2 | 21 February 2023 |
| | | | | JP | 2021182914 | A | 02 December 2021 |
| | | | | JP | 7244174 | B2 | 22 March 2023 |
| | | | | EP | 4122340 | A1 | 25 January 2023 |
| | | | | JP | 2020505063 | A | 20 February 2020 |
| | | | | JP | 6912066 | B2 | 28 July 2021 |
| | | | | EP | 4118989 | A1 | 18 January 2023 |
| | | | | JP | 2021182913 | A | 02 December 2021 |
| | | | | JP | 7248365 | B2 | 29 March 2023 |
| | | | | EP | 3571941 | A1 | 27 November 2019 |
| | | | | EP | 3571941 | B1 | 26 October 2022 |
| | | | | KR | 20180085339 | A | 26 July 2018 |
| | | | | KR | 20180085365 | A | 26 July 2018 |
| | | | | KR | 20180085367 | A | 26 July 2018 |
| | | | | KR | 20180085368 | A | 26 July 2018 |
| | | | | WO | 2018135887 | A | 26 July 2018 |
| | | | | KR | 20180085645 | A | 27 July 2018 |
| | | | | KR | 20180085647 | A | 27 July 2018 |
| | | | | JP | 2020505063 | W | 20 February 2020 |
| | | | | KR | 102185910 | B1 | 03 December 2020 |
| | | | | KR | 102187256 | B1 | 04 December 2020 |
| | | | | KR | 102199792 | B1 | 07 January 2021 |
| | | | | CN | 115005507 | A | 06 September 2022 |
| | | | | CN | 110191650 | B | 05 August 2022 |
| | | | | CN | 115153103 | A | 11 October 2022 |
| CN | 111671166 | A | 18 September 2020 | None | | | |
| CN | 109998178 | A | 12 July 2019 | WO | 2020223941 | A1 | 12 November 2020 |
| | | | | EP | 3777586 | A1 | 17 February 2021 |
| | | | | CN | 109998178 | B | 09 November 2021 |
| | | | | EP | 3777586 | B1 | 07 December 2022 |
| CN | 112471612 | A | 12 March 2021 | None | | | |
| US | 2021015165 | A1 | 21 January 2021 | WO | 2020223941 | A1 | 12 November 2020 |
| | | | | EP | 3777586 | A1 | 17 February 2021 |
| | | | | EP | 3777586 | B1 | 07 December 2022 |
| WO | 2021220384 | A1 | 04 November 2021 | TW | 202139866 | A | 01 November 2021 |
| | | | | EP | 4074200 | A1 | 19 October 2022 |
| | | | | JP | WO2021220384 | A1 | 31 July 2022 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210361280